# FASCICULE DE BREVET EUROPEEN

(11) **EP 0 554 345 B1**
(45) Date de publication et mention de la délivrance du brevet: **12.04.1995**
(21) Numéro de dépôt: 91919521.4
(22) Date de dépôt: 22.10.1991
(51) Int. Cl.: A61F 13/15

(54) **COUCHE-CULOTTE A ELEMENTS ELASTIQUES, PROCEDE ET INSTALLATION POUR SA FABRICATION EN CONTINU**
WINDELHOSE MIT ELASTISCHEN ELEMENTEN SOWIE VERFAHREN UND VORRICHTUNG ZU IHRER KONTINUIERLICHEN HERSTELLUNG
SHAPED NAPPY WITH ELASTIC CRUTCH ELEMENTS AND WAISTBAND, AND PROCESS AND PLANT FOR ITS CONTINUOUS MANUFACTURE

(30) Priorité: 26.10.1990 FR 9013268
(43) Date de publication de la demande: 11.08.1993
(73) Titulaire: PEAUDOUCE, 59126 Linselles (FR)
(72) Inventeur: PFEIFER, Roland, F-59910 Bondues (FR); BERNTSSON, Joakim, S-439 00 Onsala (SE)
(74) Mandataire: Casalonga, Axel
(86) Numéro de dépôt international: FR9100830
(87) Numéro de publication internationale: WO9207531

(56) Documents cités:
- EP-A- 0 048 010
- EP-A- 0 048 011
- EP-A- 0 121 178
- WO-A-86/02530
- DE-A- 3 613 086
- US-A- 4 618 384
- US-A- 4 726 873

## Description

La présente invention se rapporte à des couches-culottes à jeter après usage ainsi qu'à un procédé et à une installation pour la fabrication en continu de telles couches-culottes.

De telles couches-culottes qui peuvent être utilisées pour des enfants en bas âge ou pour des personnes adultes incontinentes comprennent habituellement une feuille extérieure imperméable aux liquides, une feuille intérieure perméable aux liquides, les deux feuilles ayant une forme généralement sensiblement rectangulaire avec de préférence des échancrures médianes découpées dans leurs bords longitudinaux pour permettre le passage des jambes et définissant dans le sens de la longueur de la couche-culotte une zone d'entrejambes plus étroite entre deux zones d'extrémité ou de ceinture plus larges. Ces couches-culottes comprennent, en outre, un coussin absorbant ayant des dimensions inférieures à celles desdites deux feuilles et disposé entre lesdites feuilles de manière que ses bords se trouvent en retrait par rapport aux bords correspondant des deux feuilles, lesdites deux feuilles étant reliées entre elles par collage, le long de leurs bords autour du coussin absorbant. Ces couches-culottes comprennent, par ailleurs, des éléments d'attache pour fermer la couche-culotte, par les deux zones d'extrémité, autour de la taille de l'utilisateur. Ces attaches peuvent être par exemple des attaches adhésives disposées sur les bords longitudinaux des feuilles, de préférence au voisinage du bord transversal de la zone d'extrémité formant la partie arrière de la couche-culotte.

Dans le but d'améliorer l'étanchéité au passage des jambes, il est par ailleurs connu de prévoir, sur de telles couches-culottes, des éléments élastiques d'entrejambes collés sous tension sur la face interne de la feuille extérieure imperméable, de chaque côté du coussin absorbant, le long de chacune desdites échancrures.

Ces éléments élastiques d'entrejambes sont généralement rectilignes, mais il a également déjà été proposé par la demande de brevet EP-A-048 010 de fixer ces éléments élastiques sous une forme curviligne pour leur permettre de suivre la forme des échancrures. Suivant ce document, les éléments élastiques sont fixés sous tension à la feuille imperméable uniquement dans la zone d'entrejambes.

Il a par ailleurs été proposé par la demande de brevet FR-A-2 542 979 de fixer de tels éléments élastiques longitudinaux sous tension variable à une couche-culotte, sur toute la longueur de cette dernière, à savoir sous forte tension et sous une forme curviligne dans la zone d'entrejambes et sous une tension moindre dans les zones d'extrémité.

En outre, la demande de brevet WO-A-86/02530 (&EP-A-229753) décrit une couche-culotte à jeter après usage comprenant une feuille extérieure imperméable aux liquides, une feuille intérieure perméable aux liquides, les deux feuilles ayant une forme générale sensiblement rectangulaire avec des échancrures médianes opposées découpées dans leurs bords longitudinaux pour permettre le passage des jambes et définissant dans le sens de là longueur de la couche-culotte une zone d'entrejambes plus étroite entre deux zones d'extrémité ou de ceinture plus larges, un coussin absorbant ayant des dimensions inférieures à celles desdites deux feuilles, disposé entre lesdites deux feuilles de manière que ses bords se trouvent en retrait par rapport aux bords correspondants desdites deux feuilles, lesdites deux feuilles étant reliées entre elles par collage sur leurs bords, autour du coussin absorbant, des éléments d'attache pour fermer la couche-culotte par es deux zones d'extrémité autour de la taille de l'utilisateur, et des éléments élastiques fixés sous tension par collage à la face interne de ladite feuille imperméable, de part et d'autre du coussin absorbant, sensiblement sur toute la longueur de la feuille imperméable, avec une tension plus forte dans la zone d'entrejambes que dans les zones d'extrémité, de manière à présenter dans les deux zones d'extrémité des tronçons convergeant en direction des bords transversaux de la feuille imperméable.

Ces élastiques améliorent l'étanchéité latérale de la couche-culotte, mais ne contribuent en aucune manière à l'amélioration de l'étanchéité dans le sens longitudinal (à l'endroit de la ceinture).

Enfin, on connaît par la demande de brevet GB-A-2 173 689 une couche-culotte comportant à la fois des élastiques longitudinaux s'étendant sur toute la longueur de la couche-culotte, de part et d'autre du coussin absorbant, et des éléments élastiques transversaux s'étendant sur toute la largeur de la couche-culotte dans les deux zones d'extrémité. L'inconvénient principal de l'utilisation de ces deux types d'éléments élastiques consiste dans les difficultés de fabrication en continu de telles couches-culottes lorsque les éléments élastiques doivent être fixés à l'état tendu à la feuille imperméable. En effet, de telles couches-culottes sont fabriquées en continu, soit dans le sens longitudinal, soit dans le sens transversal, à partir d'une bande continue de feuille imperméable sur laquelle soit les éléments élastiques longitudinaux, soit les éléments élastiques transversaux sont fixés en continu à l'état tendu. Par contre, la fixation sous tension des autres éléments élastiques s'étendant perpendiculairement à la direction de fabrication ne peut s'effectuer en continu, ce qui limite considérablement les cadences de fabrication. De plus, l'utilisation, sur chaque couche-culotte, de deux éléments élastiques longitudinaux et de deux éléments élastiques transversaux augmente le coût des couches-culottes.

La présente invention a pour objet une couche-culotte qui, tout en étant d'une fabrication simple et d'un coût réduit, assure à la fois une bonne étanchéité dans la zone d'entrejambes et dans les zones de ceinture. L'invention a également pour objet un procédé pour la fabrication en continu de telles couches-culottes, procédé qui soit particulièrement simple et admette des cadences de fabrication particulièrement élevées. L'invention a par ailleurs pour objet une installation pour la mise en oeuvre de ce procédé, conçue de manière à permettre d'une part une grande cadence de fabrication et d'autre part une pose particulièrement précise des éléments élastiques.

La couche-culotte à jeter après usage conforme à l'invention comprend une feuille extérieure imperméable aux liquides, une feuille intérieure perméable aux liquides, les deux feuilles ayant une forme générale sensiblement rectangulaire avec des échancrures médianes découpées sur leurs bords longitudinaux pour permettre le passage des jambes et définissant dans le sens de la longueur de la couche-culotte une zone d'entrejambes plus étroite entre deux zones d'extrémité ou ceinture plus larges. La couche-culotte comprend, en outre, un coussin absorbant ayant des dimensions inférieures à celles des deux feuilles, disposé entre lesdites deux feuilles de manière que ses bords se trouvent en retrait par rapport aux bords correspondants des deux feuilles, lesdites deux feuilles étant reliées entre elles par collage sur leurs bords, autour du coussin absorbant. La couche-culotte comprend, par ailleurs, des éléments d'attache pour fermer la couche-culotte par les deux zones d'extrémité autour de la taille de l'utilisateur. La couche-culotte comprend, enfin, des éléments élastiques fixés sous tension par collage à la face interne de la feuille imperméable, de part et d'autre du coussin absorbant, sensiblement sur toute la longueur de la feuille imperméable, avec une tension plus forte dans la zone d'entrejambes que dans les zones d'extrémité. Selon l'invention, les éléments élastiques disposés sur les côtés opposés du coussin absorbant présentent, dans deux zones d'extrémité, des tronçons convergeant en direction des bords transversaux de la feuille imperméable, lesdits tronçons convergents se rapprochant l'un de l'autre vers chaque bord transversal de la couche-culotte jusqu'à une distance réciproque inférieure à 50 % de la largeur maximale du coussin absorbant, de telle manière que les éléments élastiques constituent une barrière élastique pratiquement fermée autour du coussin absorbant et procurent à la feuille imperméable à la fois une élasticité longitudinale dans la zone d'entrejambes et une élasticité transversale dans les zones d'extrémité.

Sur la couche-culotte conforme à l'invention, il est donc possible d'obtenir, à l'aide de deux éléments élastiques seulement, un résultat équivalent à celui obtenu jusqu'à présent par quatre éléments élastiques.

De préférence, les tronçons convergents des éléments élastiques présentent un tracé sensiblement rectiligne et sont inclinés d'au moins 45° et de préférence d'au moins 60° par rapport à la direction longitudinale de la couche-culotte.

Par ailleurs, la longueur des tronçons convergents des éléments élastiques est de préférence telle que la longueur des projections desdits tronçons sur les bords transversaux de la couche-culotte corresponde à au moins 40 % et de préférence à plus de 50 % de la longueur desdits bords transversaux de la couche-culotte.

Par ailleurs, sur la couche-culotte conforme à l'invention, les tronçons convergents des deux éléments élastiques se rejoignent ou se rapprochent le plus possible l'un de l'autre vers chaque bord transversal de la couche-culotte. La distance maximale séparant les deux éléments élastiques à l'endroit ou à proximité des bords transversaux de la couche-culotte correspond à moins de 20 % et de préférence à moins de 15 % de la longueur desdits bords, c'est-à-dire de la largeur de la couche-culotte à l'endroit des bords transversaux. Selon la forme du matelas absorbant, cette distance est également inférieure à 50 % et de préférence inférieure à 30 % de la largeur maximale du coussin absorbant.

Les éléments élastiques présentent avantageusement, dans les tronçons convergents, une tension qui est d'au moins 50% inférieure à la tension des éléments élastiques dans la zone d'entrejambes.

Dans le cas de couches-culottes comportant un coussin absorbant ayant, tout comme les deux feuilles, une forme en sablier, c'est-à-dire une forme générale rectangulaire avec deux échancrures latérales opposées dans la zone d'entrejambes, il est avantageux que les éléments élastiques présentent, dans la partie médiane de la zone d'entrejambes, des tronçons sensiblement rectilignes suivis des deux côtés de tronçons incurvés concaves vers l'extérieur eux-mêmes suivis dans les zones d'extrémité de tronçons incurvés convexes vers l'extérieur auxquels se raccordent lesdits tronçons convergents. Dans ce cas, lesdits tronçons convexes vers l'extérieur présentent avantageusement une tension plus faible que les tronçons convergents ayant eux-mêmes une tension inférieure à la tension des éléments élastiques dans la zone d'entrejambes.

Ainsi, les éléments élastiques peuvent par exemple être allongés entre environ 100 et 200% dans la zone d'entrejambes, entre environ 5 et 10% dans les tronçons convexes vers l'extérieur et entre environ 40 et 60% dans les tronçons convergents.

Dans le cadre de l'invention, chaque élément élastique peut, soit être constitué par un brin élastique, soit comprendre une pluralité de brins élastiques individuels disposés parallèlement entre eux.

Suivant le procédé conforme à l'invention de fabrication en continu de telles couches-culottes, on fait passer une feuille imperméable continue sur un élément de support et d'entraînement. Pour fixer des éléments élastiques par collage sur ladite feuille, on amène des éléments élastiques continus en contact avec ladite feuille, sur ledit élément, en faisant varier périodiquement la tension desdits éléments élastiques à leurs points de contact avec ladite feuille. On déplace périodiquement lesdits points de contact transversalement à la direction longitudinale de ladite feuille de façon à fixer lesdits éléments élastiques par collage sur ladite feuille de telle manière que lesdits éléments élastiques forment, sur ladite feuille, deux profils continus répétitifs, symétriques par rapport à l'axe longitudinal de la feuille. Suivant l'invention, on commande le déplacement transversal périodique des points de contact entre les éléments élastiques et la feuille imperméable de telle manière que lesdits deux profils périodiquement se croisent ou se rapprochent l'un de l'autre jusqu'à une distance réciproque inférieure à 50 % de la largeur maximale des coussins absorbants.

Pour simplifier la fabrication, il est avantageux non pas d'encoller les éléments élastiques, mais d'encoller la feuille imperméable sur sa face recevant les éléments élastiques, en vue du collage de ces derniers sur la feuille.

Afin de permettre, lors du découpage transversal subséquent de la feuille imperméable et des éléments élastiques collés sur cette dernière une rétraction des extrémités des éléments élastiques, il est possible de réserver, à l'encollage, une bande non encollée au milieu de la largeur de la feuille imperméable.

Dans le même but, il est possible d'interrompre par intermittence l'encollage de la feuille imperméable dans une zone située au milieu de la largeur de ladite feuille, de manière à former périodiquement une fenêtre non encollée à l'endroit où les trajets des éléments élastiques se croisent ou se rapprochent.

Toujours dans le même but, il est également possible d'encoller la feuille imperméable en continu sur toute sa largeur et de pulvériser périodiquement un matériau anti-adhésif sur les éléments élastiques.

L'installation pour la fabrication en continu de couches-culottes suivant la présence invention comprend, pour le guidage précis de chaque élément élastique, un moyen de guidage monté, à proximité immédiate de l'élément de support et d' entraînement sur lequel la feuille imperméable passe pour recevoir les éléments élastiques, de façon librement orientable autour d'un axe sensiblement perpendiculaire à la surface dudit élément, de préférence par un palier anti-friction, sur un chariot entraîné en translation transversalement à la direction de déplacement de l'élément de support et d'entraînement. Ce chariot peut être entraîné par un moteur linéaire ou rotatif de préférence commandé avec indexation sur le mouvement de l'élément de support et d'entraînement par une unité de contrôle électronique, gérée par ordinateur.

Ledit moyen de guidage peut avantageusement comprendre une tête de guidage avec des moyens de maintien fermés pour les éléments élastiques, par exemple sous la forme de perçages ou de canaux parallèles dans lesquels les éléments élastiques sont enfilés, de sorte que les éléments élastiques ne risquent pas de s'échapper des moyens de maintien pendant les mouvements de translation du moyen de guidage.

En outre, les tensions variables d'étirage des éléments élastiques peuvent être produites par des organes d'alimentation rotatifs qui exercent un effet de pincement sur les éléments élastiques et dont l'entraînement à vitesse variable est commandé par la même unité de contrôle électronique gérée par ordinateur.

De préférence, les trajectoires de positionnement des éléments élastiques sur la feuille imperméable peuvent être tracées et gérées par un programme d'ordinateur, de même que la forme des échancrures d'entrejambes découpées dans la feuille imperméable, par exemple par un dispositif à jets d'eau, permettant un adjustement précis du bord externe des éléments élastiques par rapport au bord des échancrures.

En se référant aux dessins annexés, on va décrire ci-après plus en détail plusieurs modes de réalisation illustratifs et non limitatifs de l'invention; sur les dessins :
la figure 1 est une vue schématique de côté des principaux éléments d'une installation pour la fabrication en continu de couches-culottes conformes à l'invention;
la figure 2 est une vue de dessus d'une partie seulement des moyens d'alimentation, de tension et de guidage des éléments élastiques de la figure 1;
la figure 3 est une vue schématique de côté d'une variante de l'installation de la figure 1;
la figure 4 est une vue illustrant plusieurs étapes de fabrication d'un premier mode de réalisation de couches-culottes selon l'invention;
la figure 5 est une vue de dessus partiellement arrachée d'une couche-culotte fabriquée selon la figure 4;
la figure 6 est une vue illustrant plusieurs étapes de fabrication d'un deuxième mode de réalisation de couches-culottes selon l'invention;
la figure 7 est une vue de dessus partiellement arrachée d'une couche-culotte fabriquée selon la figure 6.

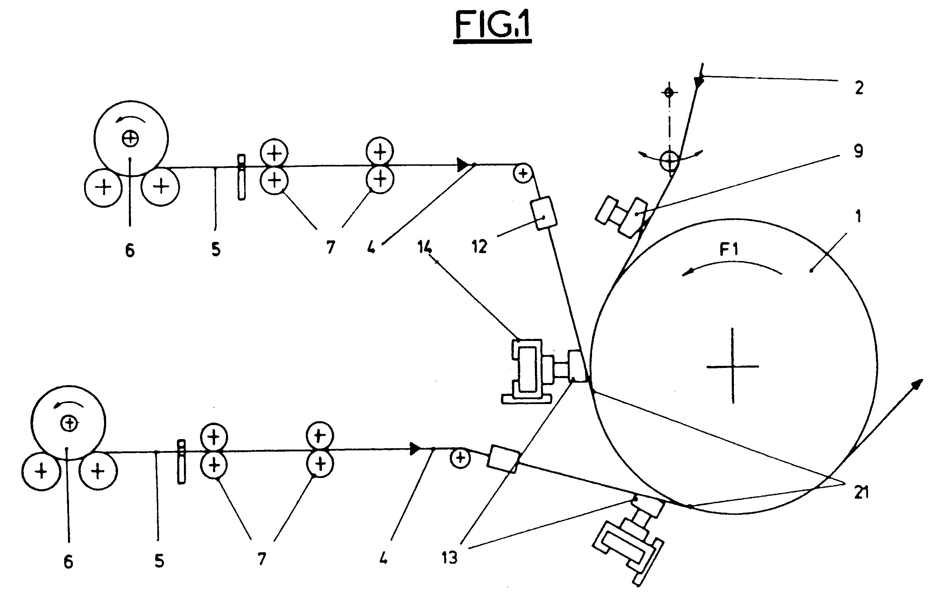

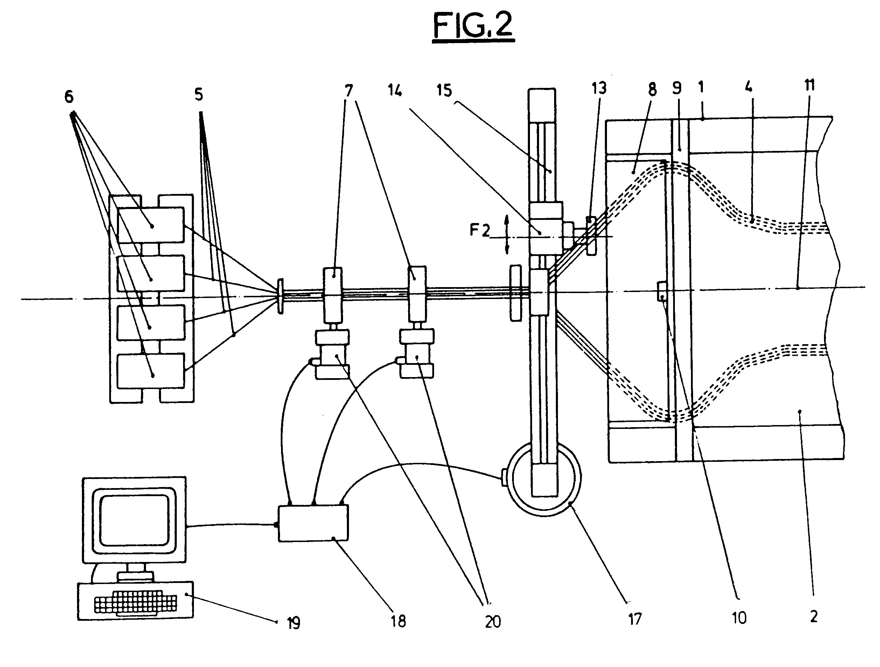

Telle qu'elle est représentée de manière très schématique sur les figures 1 et 2, l'installation pour la fabrication en continu de couches-culottes selon l'invention comprend un tambour rotatif 1 sur lequel vient s'enrouler une feuille souple continue 2 d'un matériau thermosoudable imperméable à l'humidité tel que du polyéthylène. Le tambour 1 est entraîné en rotation dans le sens de la flèche F1 de sorte que la feuille 2 épouse sur environ la moitié de sa périphérie le tambour rotatif 1. Ce dernier est maintenu à une température relativement basse comprise entre 30°C et 100°C, obtenue éventuellement par un refroidissement artificiel forcé à l'intérieur du tambour.

Deux groupes ou faisceaux 4 de quatre brins élastiques individuels parallèles 5, pouvant être par exemple des fils élastiques du type LYCRA ou des fils texturés, ou tout autre matériau élastomère approprié, dévidés à partir de bobines 6, sont entraînés entre deux groupes 7 de rouleaux tendeurs, entraînés en rotation de façon que la vitesse de déplacement linéaire des brins élastiques 5 à la sortie des rouleaux tendeurs 7 soit inférieure à la vitesse périphérique de la feuille imperméable 2 sur le tambour rotatif 1.

En réglant la différence de vitesse de manière convenable, on obtient l'élongation désirée des brins élastiques 5, que l'on peut faire varier périodiquement au cours du défilement de ceux-ci.

Un matériau adhésif 8 rendu liquide par fusion (de type hot-melt) est délivré par un dispositif d'encollage du type buse 9 qui l'extrude en continu sur toute la largeur de la feuille imperméable 2 qui défile sous cette buse 9, à l'exception d'une zone centrale 10, de surface réduite, située sur l'axe longitudinal 11 de ladite feuille 2, et qui se répète selon un pas correspondant à la longueur de la future couche-culotte à former.

Les quatre brins élastiques 5 de chaque faisceau 4 passent ensuite sur un premier organe de guidage fixe 12 puis sur une tête de guidage 13 pourvue de perçages ou canaux pour guider et maintenir les brins parallèles entre eux, montée à rotation libre avec palier sans frottement par exemple du type à coussin d'air sur un chariot mobile 14 pouvant coulisser sur une glissière 15, selon les directions indiquées par la double flèche F2.

Le chariot est entrainé par un moteur rotatif 17 ou éventuellement par un moteur linéaire commandé en fonction de la rotation du tambour 1 donc de la position de défilement de la feuille imperméable 2, par une unité de contrôle électronique 18, pilotée par un programme d'ordinateur 19. Ce même dispositif de contrôle électronique permet de commander les moteurs 20 actionnant les rouleaux tendeurs 7.

Après leur passage sur les têtes de guidage 13 (dont une seule est représentée sur la figure 2), les brins élastiques viennent en contact en 21 avec la feuille imperméable 2 sur sa face encollée, à la périphérie du tambour rotatif 1, les têtes de guidage étant disposées à proximité immédiate du point de contact 21. Les brins élastiques 5 se dirigent, entre la tête de guidage 13 et le point de contact 21 avec la feuille imperméable se trouvant à la périphérie du tambour rotatif 1, selon une direction tangente par rapport au profil du tambour rotatif 1.

La rotation sans frottement de la tête de guidage 13 autour d'un axe perpendiculaire à l'axe du tambour 1, c'est-à-dire dans un plan tangent à la surface périphérique du tambour 1 dans la zone du point de contact 21, permet de maintenir en permanence l'orientation des brins élastiques à l'entrée de la tête de guidage, dans l'axe de la trajectoire selon laquelle lesdits brins sont collés sur la feuille imperméable 2, leur évitant de subir des efforts tranversaux importants nuisibles à leur bon positionnement et à leur collage correct sur ladite feuille imperméable 2.

Les caractéristiques de l'installation, en particulier du tambour rotatif, et la nature de l'adhésif sont optimisées afin qu'un collage immédiat des brins élastiques sur la feuille imperméable intervienne aussitôt après les points de contact 21, permettant le collage des brins élastiques selon un double profil curviligne, symétrique par rapport à l'axe longitudinal (11), tel que visible sur les figures suivantes.

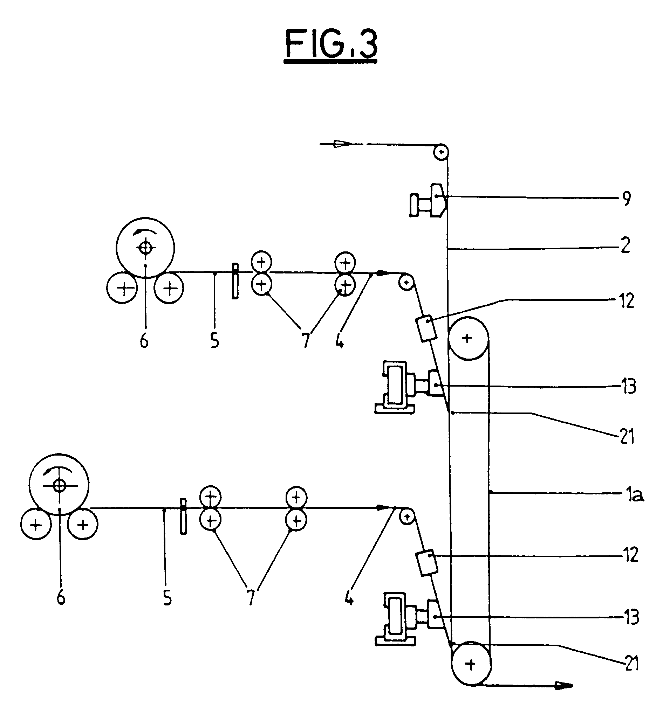

L'installation selon la figure 3 reprend la plupart des éléments de l'installation de la figure 1, à cette différence près que le tambour rotatif 1 servant d'élément de support et d'entraînement pour la feuille imperméable 2 est ici remplacé par un tapis ou une bande transporteuse 1a sans fin, rotative, de préférence aspirante, entraînant la feuille 2 à plat.

La figure 2 et la description donnée à ce sujet s'appliquent également à l'installation de la figure 3, en tenant compte du fait que la bande 1a entraîne la feuille 2 suivant un mouvement de translation et non pas suivant un mouvement de rotation comme un tambour.

Le principal avantage de l'utilisation d'une bande transporteuse 1a aspirante, au lieu d'un tambour rotatif, consiste dans le positionnement plus précis des brins élastiques 5 de chaque faisceau 4 sur la feuille imperméable 2. En effet, sur un tambour, la feuille imperméable 2 subit, après application des brins élastiques sous tension, une rétraction transversale provoquant une ondulation de la feuille, c'est-à-dire une déformation qui nuit à la position parallèle des brins dans le faisceau. De plus, du fait de la courbure de la surface du tambour, la distance entre les différents brins d'un faisceau varie à la dépose de ces brins sur la feuille imperméable 2; au cours des mouvements transversaux de la tête de guidage 13, c'est-à-dire lors du pivotement de cette tête autour de son axe de rotation. Au contraire, lors de la dépose des brins élastiques 5 sur une feuille imperméable 2 supportée et entraînée à plat par une bande transporteuse 1a aspirante, cette feuille est parfaitement maintenue jusqu'à la fixation définitive des brins élastiques et ne peut donc pas se déformer, et les conditions géométriques dans lesquelles se fait la dépose des brins élastiques sur la feuille 2 transportée à plat par la bande 1a assurent une distance invariable entre les brins, c'est-à-dire le parallélisme des brins, sur la feuille imperméable 2.

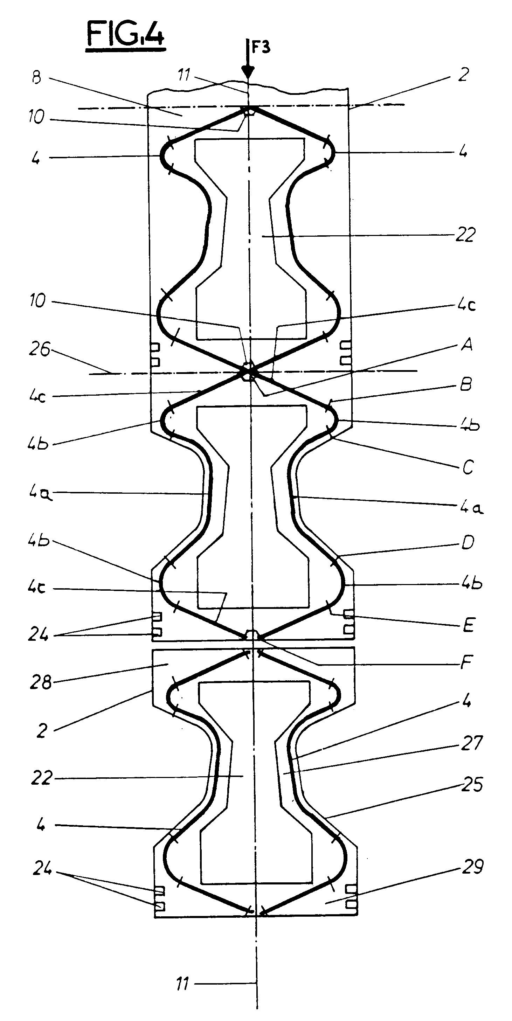

Dans un premier mode de réalisation illustré sur la figure 4, la feuille imperméable continue 2, qui se déroule dans le sens de la flèche F3 disposée selon l'axe longitudinal 11 de ladite feuille 2, et qui a reçu une enduction d'adhésif 8, reçoit le long de ses deux bords longitudinaux, deux faisceaux 4 de quatre brins élastiques individuels parallèles continus représentés pour plus de clarté par un seul trait épais. Ces deux faisceaux se croisent à intervalle régulier en formant un motif répétitif de contour fermé dont la longueur selon l'axe 11 correspond à celle des couches-culottes.

Les portions curvilignes concaves 4a comprises entre les points C et D, correspondant à la zone centrale d'entrejambes des futures couches, sont collées à l'état fortement étiré selon le procédé qui a été décrit en référence aux figures 1, 2 et 3, avec une valeur de l'allongement des brins élastiques proche de 180%, tandis que les portions curvilignes convexes 4b, comprises entre les points Det E, et B et C, n'ont subi qu'un étirement très faible, correspondant à un allongement de seulement 5 à 10%; enfin, les portions rectilignes 4c comprises entre les points A et B, et E et F qui relient les portions curvilignes précédentes 4b de deux motifs successifs, ont été étirées un peu plus fortement, de sorte que les brins élastiques présentent ici un allongement d'environ 50%.

Il faut noter que ces portions rectilignes 4c des brins élastiques se croisent selon un X, en une zone 10 centrée sur l'axe longitudinal 11 de la feuille 2, et qui est dépourvue de matériau adhésif, de sorte qu'à cet endroit, les brins élastiques ne sont pas fixés à la feuille imperméable 2.

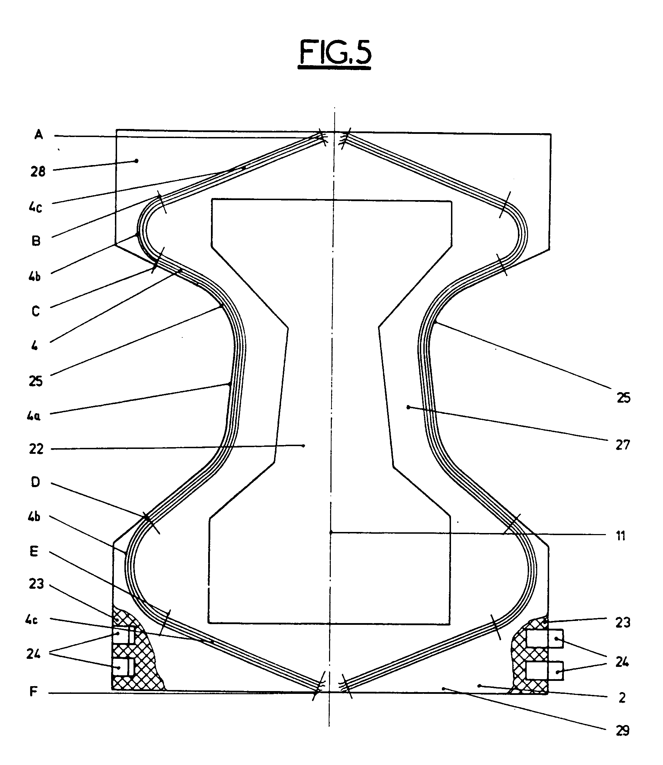

Dans une étape ultérieure du procédé, a lieu la dépose de coussins absorbants 22 de forme anatomique ou de toute autre forme, ainsi que d'un voile perméable à l'humidité 23 (non représenté sur la figure 4, mais visible sur la figure 5), qui sont collés sur la feuille imperméable 2 par le matériau adhésif 8, puis d'attaches adhésives 24 sur les bords longitudinaux de la feuille 2. Après l'étape de découpe des échancrures 25, on procède au stade final de fabrication consistant à découper transversalement selon les lignes 26 la bande composite obtenue de façon à former les couches-culottes telles que celle qui est illustrée sur la figure 5.

Lors de la découpe transversale, les portions rectilignes 4c des brins élastiques sont sectionnées au niveau de leur croisement et leurs extrémités se rétractent sur une petite longueur correspondant à la zone 10 dépourvue de matériau adhésif 8, c'est-à-dire jusqu'aux points A et F.

On obtient finalement, comme on peut le voir sur la figure 5, une couche-culotte dans laquelle les échancrures 25 d'entrejambes présentent un profil curviligne concave, bordé intérieurement d'un ensemble 4 de brins élastiques curvilignes posés à l'état fortement tendu, dont le brin le plus externe est disposé à proximité du profil de l'échancrure, assurant une étanchéité convenable contre les risques de fuites transversales du coussin absorbant à cet endroit. Les deux ensembles de brins élastiques 4 s'étendent de façon continue et symétrique par rapport à l'axe longitudinal 11, en formant un contour de périmètre pratiquement fermé autour du coussin absorbant 22. Ces deux ensembles de brins élastiques permettent donc à la fois d'assurer l'élasticité longitudinale dans la zone d'entrejambes 27 et de constituer aussi dans les zones d'extrémité avant 28 et arrière 29 de la couche-culotte des ceintures élastifiées qui facilitent l'ajustement à la taille de l'utilisateur et limitent fortement les risques de fuite le long des bords transversaux de la couche-culotte.

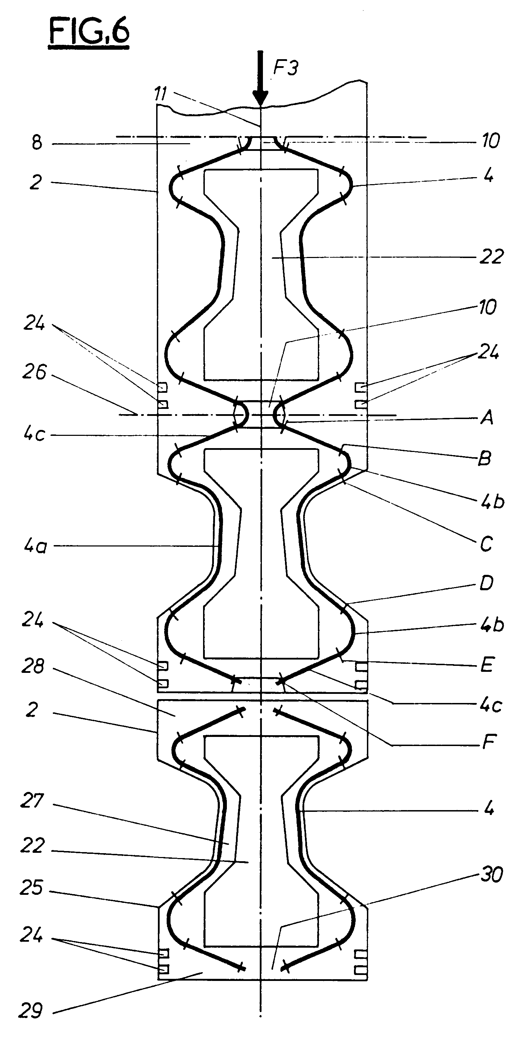

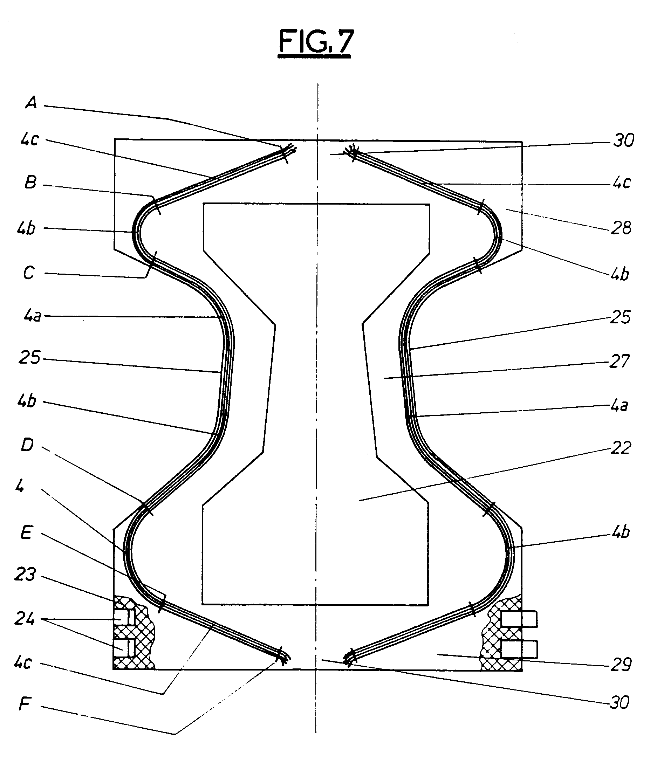

Dans un deuxième mode de réalisation illustré sur les figures 6 et 7 où les éléments identiques portent les mêmes références, la différence essentielle par rapport au mode de réalisation précédent réside dans le fait que les deux faisceaux 4 de brins élastiques individuels parallèles continus sont déposés sur la feuille imperméable 2 défilant selon la flèche F3, le long de ses bords longitudinaux, sans se croiser à intervalles réguliers.

Il y a rapprochement des portions rectilignes 4c symétriques par rapport à l'axe 11, jusqu'aux points A, mais ensuite les têtes de guidage des deux faisceaux 4 de brins élastiques, qui peuvent d'ailleurs être montées sur des chariots coulissant sur la même glissière, sont entraînées en sens contraire, et les faisceaux 4 s'écartent à partir des points F. Entre les points A et F de deux motifs successifs, les brins élastiques se trouvant appliqués sur la feuille 2 dans les zones 10 dépourvues de matériau adhésif, pourront se rétracter lors de l'étape de découpe transversale selon les lignes 26.

On obtient donc, comme représenté sur la figure 7, une couche-culotte très voisine de celle de la figure 5, mais dont les deux ensembles 4 de brins élastiques forment un contour élastifié autour du coussin absorbant dont le périmètre présente, aux deux extrémités (28 et 29) de la couche-culotte selon son axe longitudinal 11, une ouverture 30 dont la largeur peut être ajustée, en fonction des commandes de déplacement des têtes de guidage des brins élastiques et/ou de la surface de la zone 10 non adhésive, éventuellement à une valeur extrêmement réduite de façon à se rapprocher du mode de réalisation des figures 4 et 5.

Il est à noter que la "fenêtre" ou zone non adhésive 10 sur la feuille imperméable 2 peut avantageusement être remplacée par la pulvérisation d'une solution anti-adhésive sur les parties d'extrémité des portions rectilignes 4c des brins élastiques, dans tous les modes de réalisation envisagés.

Pour empêcher les extrémités des éléments élastiques 4 d'adhérer à la feuille imperméable 2, il serait également possible de réserver, à l'encollage 8 de cette feuille, une bande non encollée au milieu de la largeur de la feuille imperméable, sur toute la longueur de cette derrière. Cela permettrait notamment de positionner, directement sur la face intérieure de la feuille imperméable, un indicateur d'humidité, par exemple par une impression à l'aide d'encre soluble dans l'eau.

Par ailleurs, bien que dans les exemples illustrés, les différentes portions de tension variable des brins élastiques aient les valeurs déterminées ci-dessus, on comprendra que l'invention ne soit pas limitée à l'utilisation de telles valeurs précises. En outre on pourrait envisager sans modification importante, de réaliser une variation continue de la tension des éléments élastiques sur toute la longueur de chaque couche-culotte.

Enfin, dans le cadre de l'invention, le coussin absorbant, au lieu d'avoir une forme anatomique en sablier, pourrait également avoir par exemple une forme rectangulaire, sans échancrures latérales, auquel cas les éléments élastiques pourraient avoir un tracé sensiblement rectiligne sur toute la zone d'entrejambes.

## Revendications

1. Couche-culotte à jeter après usage comprenant une feuille extérieure (2) imperméable aux liquides, une feuille intérieure (23) perméable aux liquides, les deux feuilles ayant une forme générale sensiblement rectangulaire avec des échancrures médianes (25) opposées découpées dans leurs bords longitudinaux pour permettre le passage des jambes et définissant dans le sens de la longueur de la couche-culotte une zone d'entrejambes (27) plus étroite entre deux zones d'extrémité ou de ceinture (28, 29) plus larges, un coussin absorbant (22) ayant des dimensions inférieures à celles desdites deux feuilles, disposé entre lesdites deux feuilles de manière que ses bords se trouvent en retrait par rapport aux bords correspondants desdites dieux feuilles, lesdites deux feuilles étant reliées entre elles par collage sur leurs bords, autour du coussin absorbant, des éléments d'attache (24) pour fermer la couche-culotte par les deux zones d'extrémité (28, 29) autour de la taille de l'utilisateur, et des éléments élastiques (4) fixés sous tension par collage à la face interne de ladite feuille imperméable (2), de part et d'autre du coussin absorbant (22), sensiblement sur toute la longueur de la feuille imperméable, avec une tension plus forte dans la zone d'entrejambes (27) que dans les zones d'extrémité (28, 29), de manière à présenter dans les deux zones d'extrémité (28, 29) des tronçons (4c) convergeant en direction des bords transversaux de la feuille imperméable, caractérisée par le fait que les tronçons convergents (4c) des éléments élastiques (4) se rapprochent l'un de l'autre vers chaque bord transversal de la couche-culotte jusqu'à une distance réciproque inférieure à 50% de la largeur maximale du coussin absorbant (22), ce telle manière que les éléments élastiques (4) constituent une barrière élastique pratiquement fermée autour du coussin absorbant (22) et procurent à la feuille imperméable à la fois une élasticité longitudinale dans la zone d'entrejambes et une élasticité transversale dans les zones d'extrémité.

2. Couche-culotte suivant la revendication 1, caractérisée par le fait que les tronçons convergents (4c) des éléments élastiques (4) présentent un tracé sensiblement rectiligne et sont inclinés d'au moins 45° et de préférence d'au moins 60° par rapport à la direction longitudinale de la couche-culotte.

3. Couche-culotte suivant la revendication 1 ou 2, caractérisée par le fait que la longueur des tronçons convergents (4c) des éléments élastiques (4) est telle que la longueur des projections desdits tronçons sur les bords transversaux de la couche-culotte corresponde à au moins 40% et de préférence à plus de 50% de la longueur desdits bords transversaux de la couche-culotte.

4. Couche-culotte suivant l'une quelconque des revendications précédentes, caractérisée par le fait que les tronçons convergents (4c) des deux éléments élastique (4) se rejoignent ou se rapprochent l'un de l'autre vers chaque bord transversal de la couche-culotte jusqu'à une distance correspondant à moins de 20% et de préférence à moins de 15% de la longueur desdits bords.

5. Couche-culotte suivant l'une quelconque des revendications précédentes, caractérisée par le fait que les tronçons convergents (4c) des deux éléments élastiques (4) se rapprochent vers chaque bord transversal de la couche-culotte jusqu'à une distance réciproque inférieure à 30% de la largeur maximale du coussin absorbant (22).

6. Couche-culotte suivant l'une quelconque des revendications précédentes, caractérisée par le fait que les éléments élastiques (4) présentent, dans les tronçons convergents (4c), une tension qui est d'au moins 50% inférieure à la tension des éléments élastiques dans la zone d'entrejambes.

7. Couche culotte suivant l'une quelconque des revendications précédentes, comportant un coussin absorbant (22) de forme générale rectangulaire avec deux échancrures latérales opposées dans la zone d'entrejambes, caractérisée par le fait que les éléments élastiques (4) présentent, dans la partie médiane de la zone d'entrejambe (27), des tronçons sensiblement rectilignes suivis des deux côtés de tronçons incurvés concaves vers l'extérieur eux même suivis, dans les zones d'extrémité (28, 29), de tronçons (4b) incurvés convexes vers l'extérieur auxquels se raccordent lesdits tronçons convergents (4c), lesdits tronçons (4b) convexes vers l'extérieur présentant une tension plus faible que les tronçons convergents (4c) ayant eux mêmes une tension inférieure à la tension des éléments élastiques (4a) dans la zone d'entrejambes (27).

8. Couche-culotte suivant la revendication 7, caractérisée par le fait que les éléments élastiques (4) sont allongés entre environ 100 et 200% dans la zone à'entrejambes (27), entre environ 5 et 10% dans les tronçons (4b) convexes vers l'extérieur et entre environ 40 et 60% des les tronçons convergents (4c).

9. Couche-culotte suivant l'une quelconque des revendications précédentes, caractérisée par le fait que chaque élément élastique (4) comprend une pluralité de brins élastiques individuels disposés parallèlement entre eux.

10. Procédé de fabrication en continu de couches-culottes suivant l'une quelconque des revendications précédentes, comportant chacune une zone d'entrejambes et deux zones d'extrémité, consistant à faire passer une feuille imperméable continue sur un élément de support et d'entraînement, à amener des éléments élastiques continus en contact avec ladite feuille, sur ledit élément, en faisant varier périodiquement la tension desdits éléments élastiques à leurs points de contact avec ladite feuille, et en déplaçant périodiquement lesdits points de contact transversalement à la direction longitudinale de ladite feuille de façon à fixer lesdits éléments élastiques par collage sur ladite feuille de telle manière que lesdits éléments élastiques forment, sur ladite feuille, deux profils continus répétitifs symétriques par rapport à l'axe longitudinal de la feuille, et présentent dans les zones correspondant aux zones d'extrémité des couches-culottes une tension plus faible que dans les zones correspondant aux zones d'entrejambes des couches-culottes, à déposer sur la feuille imperméable des coussins absorbants, une feuille continue perméable aux liquides et des attaches adhésives, et à couper la bande composite ainsi obtenue transversalement entre les coussins absorbants, caractérisé par le fait qu'on commande le déplacement transversal périodique des points de contact entre les éléments élastiques et la feuille imperméable de telle manière que dans les zones correspondant aux zones d'extrémité des couches-culottes, lesdits deux profils périodiquement se croisent ou se rapprochent l'un de l'autre jusqu'à une distance réciproque inférieure à 50% de la largeur maximale des coussins absorbants.

11. Procédé suivant la revendication 10, caractérisé par le fait qu'on encolle la feuille imperméable sur sa face recevant les éléments élastiques, en vue du collage de ces derniers sur la feuille.

12. Procédé suivant la revendication 11, caractérisé par le fait qu'on réserve, à l'encollage, une bande non encollée au milieu de la largeur de la feuille imperméable.

13. Procédé suivant la revendication 11, caractérisé par le fait qu'on interrompt par intermittence l'encollage de la feuille imperméable dans une zone située au milieu de la largeur de ladite feuille, de manière à former périodiquement une fenêtre non encollée à l'endroit où les trajets des éléments élastiques se croisent ou se touchent.

14. Procédé suivant la revendication 11, caractérisé par le fait qu'on encolle la feuille imperméable en continu sur toute sa largeur et qu'on pulvérise périodiquement un matériau anti-adhésif sur les éléments élastiques.

15. Installation pour la fabrication en continu de couches-culottes suivant l'une quelconque des revendications 1 à 9, par la mise en oeuvre du procédé suivant l'une quelconque des revendications 10 à 14, caractérisée par le fait qu'eue comprend, pour le guidage de chaque élément élastique, un moyen de guidage (13) monté, à proximité immédiate de l'élément de support et d'entraînement (1,1a) sur lequel la feuille imperméable (2) passe pour recevoir les éléments élastiques (4), de façon librement orientable autour d'un axe sensiblement perpendiculaire à la surface dudit élément (1,1a), de préférence par un palier anti-friction, sur un chariot (14) entraîné en translation transversalement à la direction de déplacement de l'élément de support et d'entraînement.

16. Installation suivant la revendication 15, caractérisée par le fait que ledit chariot (14) est entraîné par un moteur (17) linéaire ou rotatif commandé avec indexation sur le mouvement de l'élément de support et d'entraînement (1) par une unité de contrôle électronique (18) gérée par ordinateur (19).

17. Installation suivant la revendication 15 ou 16, caractérisée par le fait qu'elle comprend, pour donner des tensions variables d'étirage aux éléments élastiques, des organes' d'alimentation rotatifs (7) qui exercent un effet de pincement sur les éléments élastiques et dont l'entraînement à vitesse variable est commandé par une unité de contrôle électronique (18) gérée par ordinateur (19).

18. Installation suivant l'une quelconque des revendications 16 ou 17, caractérisée par le fait que les trajectoires de positionnement des éléments élastiques sur la feuille imperméable sont tracées et gérées par un programme d'ordinateur, de même que la forme des échancrures d'entrejambes découpées dans la feuille imperméable par exemple par un dispositif à jets d'eau.

19. Installation suivant la revendication 15, caractérisée par le fait que ledit moyen de guidage (13) comprend une tête de guidage avec des moyens de maintien fermés pour les éléments élastiques, par exemple sous la forme de perçages ou de canaux parallèles dans lesquels les éléments élastiques sont enfilés.

20. Installation suivant l'une quelconque des revendications 15 à 19, caractérisée par le fait que l'élément de support et d'entraînement est constitué par un tambour rotatif (1).

21. Installation suivant l'une quelconque des revendications 15 à 19, caractérisée par le fait que l'élément de support et d'entraînement est constitué par une bande transporteuse (1a) aspirante.

## Claims

1. Nappy-pants, to be disposed of after use, comprising a liquid-impervious outer sheet (2), a liquid-permeable inner sheet (23), the two sheets being of a substantially rectangular overall shape with opposed median indentations (25) cut out in their lengthwise edges to allow legs to pass through and defining in the lengthwise direction of the nappy-pants a narrower crotch region (27) between two wider end or belt regions (28, 29), an absorbent pad (22) whose dimensions are smaller than those of the said two sheets, which is arranged between the said two sheets so that its edges are set back in relation to the corresponding edges of the said two sheets, the said two sheets being joined together by adhesive bonding at their edges, around the absorbent pad, fastening members (24) for closing the nappy-pants, using the two end regions (28, 29), around the user's waist, and elastic members (4) attached under tension by adhesive bonding to the inner face of the said impervious sheet (2) on both sides of the absorbent pad (22), substantially over the whole length of the impervious sheet, with a higher tension in the crotch region (27) than in the end regions (28, 29), so as to have, in the two end regions (28, 29), sections (4c) which converge in the direction of the transverse edges of the impervious sheet, characterized in that the converging sections (4c) of the elastic members (4) approach one another near each transverse edge of the nappy-pants as far as a mutual distance of less than 50 % of the maximum width of the absorbent pad (22), so that the elastic members (4) form a practically closed elastic barrier around the absorbent pad (22) and provide the impervious sheet both with a lengthwise elasticity in the crotch region and a transverse elasticity in the end regions.

2. Nappy-pants according to Claim 1, characterized in that the converging sections (4c) of the elastic members (4) follow a substantially rectilinear path and are inclined by at least 45° and preferably at least 60° in relation to the lengthwise direction of the nappy-pants.

3. Nappy-pants according to Claim 1 or 2, characterized in that the length of the converging sections (4c) of the elastic members (4) is such that the length of the projections of the said sections onto the transverse edges of the nappy-pants corresponds to at least 40 % and preferably to more than 50 % of the length of the said transverse edges of the nappy-pants.

4. Nappy-pants according to any one of the preceding claims, characterized in that the converging sections (4c) of the two elastic members (4) join together or approach each other near each transverse edge of the nappy-pants as far as a distance corresponding to less than 20 % and preferably less than 15 % of the length of the said edges.

5. Nappy-pants according to any one of the preceding claims, characterized in that the converging sections (4c) of the two elastic members (4) approach each other near each transverse edge of the nappy-pants as far as a mutual distance of less than 30 % of the maximum width of the absorbent pad (22).

6. Nappy-pants according to any one of the preceding claims, characterized in that in the converging sections (4c), the elastic members (4) have a tension which is at least 50% lower than the tension of the elastic members in the crotch region.

7. Nappy-pants according to any one of the preceding claims, comprising an absorbent pad (22) of rectangular overall shape with two opposite side indentations in the crotch region, characterized in that the elastic members (4) have, in the median part of the crotch region (27), substantially rectilinear sections followed on both sides by outwardly concave curved sections, themselves followed in the end regions (28, 29) by outwardly convex curved sections (4b) to which the said converging sections (4c) are joined, the said outwardly convex sections (4b) having a lower tension than the converging sections (4c), themselves having a lower tension than the tension of the elastic members (4a) in the crotch region (27).

8. Nappy-pants according to Claim 7, characterized in that the elastic members (4) are extended by between approximately 100 and 200 % in the crotch region (27), between approximately 5 and 10 % in the outwardly convex sections (4b) and between approximately 40 and 60 % in the converging sections (4c).

9. Nappy-pants according to any one of the preceding claims, characterized in that each elastic member (4) comprises a plurality of individual elastic strands arranged parallel to each other.

10. Process for the continuous manufacture of nappy-pants according to any one of the preceding claims, each pair of nappy-pants including a crotch region and two end regions, consisting in passing a continuous impervious sheet over a supporting and driving member, bringing continuous elastic members into contact with the said sheet, on the said member, while varying at regular intervals the tension of the said elastic members at their points of contact with the said sheet, and moving the said points of contact at regular intervals transversely to the lengthwise direction of the said sheet so as to attach the said elastic members by adhesive bonding onto the said sheet so that the said elastic members form, on the said sheet, two repetitive continuous outlines which are symmetrical in relation to the lengthwise axis of the sheet, and having, in the regions corresponding to the end regions of the nappy-pants, a tension which is lower than in the regions corresponding to the crotch regions of the nappy-pants, in laying absorbent pads, a liquid-permeable sheet and adhesive fasteners onto the impervious sheet, and in cutting the composite strip thus obtained transversely between the absorbent pads, characterized in that the transverse movement, at regular intervals, of the points of contact between the elastic members and the impervious sheet is controlled so that in the regions corresponding to the end regions of the nappy-pants the said two outlines at regular intervals cross each other or approach each other as far as a mutual distance of less than 50 % of the maximum width of the absorbent pads.

11. Process according to Claim 10, characterized in that adhesive is applied to the impervious sheet on its face receiving the elastic members, with a view to the adhesive bonding of the latter onto the sheet.

12. Process according to Claim 11, characterized in that an adhesive-free strip is left in the middle of the width of the impervious sheet during the application of the adhesive.

13. Process according to Claim 11, characterized in that the application of adhesive to the impervious sheet is interrupted intermittently in a region situated in the middle of the width of the said sheet, so as to form at regular intervals an adhesive-free window where the paths of the elastic members cross or touch each other.

14. Process according to Claim 11, characterized in that adhesive is applied to the impervious sheet continuously over its whole width and that an antiadhesive material is sprayed at regular intervals onto the elastic members.

15. Plant for the continuous manufacture of nappy-pants according to any one of Claims 1 to 9, by implementing the process according to any one of Claims 10 to 14, characterized in that it comprises, for guiding each elastic member, a guiding means (13) mounted in the immediate proximity of the supporting and driving member (1, 1a) over which the impervious sheet (2) passes to receive the elastic members (4), so that it can be freely oriented around an axis substantially perpendicular to the surface of the said member (1, 1a), preferably by means of a frictionless bearing, on a trolley (14) driven in translation transversely to the direction of movement of the supporting and driving member.

16. Plant according to Claim 15, characterized in that the said trolley (14) is driven by a linear or rotary motor (17) controlled, with index-linking to the movement of the supporting and driving member (1), by an electronic control unit (18) commanded by a computer (19).

17. Plant according to Claim 15 or 16, characterized in that, in order to give variable stretching tensions to the elastic members, it comprises rotary feeding instruments (7) which exert a clamping effect on the elastic members and whose variable-speed drive is controlled by an electronic control unit (18) commanded by a computer (19).

18. Plant according to either of Claims 16 and 17, characterized in that the trajectories for positioning the elastic members on the impervious sheet are mapped out and controlled by a computer program, as is the shape of the crotch indentations cut out of the impervious sheet, for example by a device incorporating water jets.

19. Plant according to Claim 15, characterized in that the said guiding means (13) comprises a guiding head with closed holding means for the elastic members, for example in the form of parallel drilled holes or channels into which the elastic members are threaded.

20. Plant according to any one of Claims 15 to 19, characterized in that the supporting and driving member consists of a rotary drum (1).

21. Plant according to any one of Claims 15 to 19, characterized in that the supporting and driving member consists of a conveyor belt (1a) which applies suction.

## Patentansprüche

1. Höschenwindel zum Wegwerfen nach Gebrauch, aufweisend: eine äußere Folie (2), die für Flüssigkeiten undurchlässig ist; eine innere Folie (23), die für Flüssigkeiten durchlässig ist; wobei die beiden Folien eine im wesentlichen allgemein rechteckige Form mit im Mittelbereich befindlichen, einander gegenüberliegenden Ausschnitten (25) aufweisen, die in die Längsränder der Folien eingeschnitten sind, um den Durchgang der Beine zu ermöglichen, und die in Richtung der Länge der Höschenwindel eine Schrittzone (27) begrenzen, die zwischen zwei breiteren Endzonen oder Gürtelzonen (28, 29) eine schmalere Schrittzone (27) definieren; ein absorbierendes Kissen (22) mit Abmessungen, die kleiner als diejenigen der beiden Folien sind, wobei das Kissen zwischen den beiden Folien derart angeordnet ist, daß seine Ränder gegen die entsprechenden Ränder der beiden Folien rückgesetzt sind, wobei die beiden Folien miteinander durch Kleben entlang ihrer Ränder um das absorbierende Kissen herum verbunden sind; Befestigungselemente (24) zum Schließen der Höschenwindel mit den beiden Endzonen (28, 29) um die Hüfte des Benutzers; und elastische Elemente (4), die unter Spannung durch Ankleben an der Innenseite der undurchlässigen Folie (2) zu beiden Seiten des absorbierenden Kissens (22) im wesentlichen über die gesamte Länge der undurchlässigen Folie befestigt werden, mit einer stärkeren Spannung in der Schrittzone (27) als in den Endzonen (28, 29), derart, daß in den beiden Endzonen (28, 29) Abschnitte (4c) gebildet werden, die in Richtung der Querränder der undurchlässigen Folie konvergieren,
dadurch **gekennzeichnet,** daß sich die konvergierenden Abschnitte (4c) der elastischen Elemente (4) zu jedem Querrand der Höschenwindel hin bis auf einen gegenseitigen Abstand annähern, der kleiner als 50 % der maximalen Breite des absorbierenden Kissens (22) ist, derart, daß die elastischen Elemente (4) eine praktisch geschlossene, elastische Sperre um das absorbierende Kissen (22) herum bilden und der undurchlässigen Folie zugleich eine Längselastizität in der Schrittzone und eine Querelastizität in den Endzonen verschaffen.

2. Höschenwindel nach Anspruch 1,
dadurch **gekennzeichnet,** daß die konvergierenden Abschnitte (4c) der elastischen Elemente (4) einen im wesentlichen geradlinigen Verlauf haben und um mindestens 45°, vorzugsweise um mindestens 60°, relativ zur Längsrichtung der Höschenwindel, geneigt sind.

3. Höschenwindel nach Anspruch 1 oder 2,
dadurch **gekennzeichnet,** daß die Länge der konvergierenden Abschnitte (4c) der elastischen Elemente (4) so bemessen ist, daß die Länge der Projektionen dieser Abschnitte auf die Querränder der Höschenwindel mindestens 40 %, und vorzugsweise mehr als 50 % der Länge der Querränder der Höschenwindel entspricht.

4. Höschenwindel nach irgendeinem vorhergehenden Anspruch,
dadurch **gekennzeichnet,** daß die konvergierenden Abschnitte (4c) der beiden elastischen Elemente (4) wieder zusammenkommen oder sich einander zu jedem Querrand der Höschenwindel hin annähern, bis auf einen Abstand, der mindestens 20 %, und vorzugsweise mindestens 15 % der Länge der genannten Ränder ausmacht.

5. Höschenwindel nach irgendeinem vorhergehenden Anspruch,
dadurch **gekennzeichnet,** daß sich die konvergierenden Abschnitte (4c) der beiden elastischen Elemente (4) nach jedem Querrand der Höschenwindel hin bis auf einen gegenseitigen Abstand hin annähern, der kleiner als 30 % der maximalen Breite des absorbierenden Kissens (22) ist.

6. Höschenwindel nach irgendeinem vorhergehenden Anspruch,
dadurch **gekennzeichnet,** daß die elastischen Elemente (4) in den konvergierenden Abschnitten (4c) eine Spannung aufweisen, die um mindestens 50 % niedriger als die Spannung der elastischen Elemente in der Schrittzone ist.

7. Höschenwindel nach irgendeinem vorhergehenden Anspruch, mit einem absorbierenden Kissen (22) von allgemein rechteckiger Form, mit zwei seitlichen, einander gegenüberliegenden Einschnitten in der Schrittzone,
dadurch **gekennzeichnet,** daß die elastischen Elemente (4) im Mittelabschnitt der Schrittzone (27) Abschnitte aufweisen, die im wesentlichen geradlinig verlaufen und an die sich zu beiden Seiten einwärtsgekrümmte, nach außen hin konkave Abschnitte anschließen, an die sich ihrerseits in den Endzonen (28, 29) einwärtsgekrümmte, nach außen hin konvexe Abschnitte (4b) anschließen, auf die die genannten konvergierenden Abschnitte (4c) folgen, wobei die nach außen hin konvexen Abschnitte (4b) eine geringere Spannung aufweisen als die konvergierenden Abschnitte (4c), die ihrerseits eine geringere Spannung als die Spannung der elastischen Elemente (4a) in der Schrittzone (27) aufweisen.

8. Höschenwindel nach Anspruch 7,
dadurch **gekennzeichnet,** daß die elastischen Elemente (4) zwischen 100 und 200 % in der Schrittzone (27), zwischen ungefähr 5 und 10 % in den nach außen hin konvexen Abschnitten (4b), und zwischen ungefähr 40 und 60 % in den konvergierenden Abschnitten (4c), gedehnt sind.

9. Höschenwindel nach irgendeinem vorhergehenden Anspruch,
dadurch **gekennzeichnet,** daß jedes elastische Element (4) eine Vielzahl von einzelnen, elastischen Fäden umfaßt, die parallel zueinander angeordnet sind.

10. Verfahren zu Herstellung von Höschenwindeln in kontinuierlicher Folge nach irgendeinem vorhergehenden Anspruch, wobei jede Windel eine Schrittzone und zwei Endzonen aufweist, und wobei das Verfahren darin besteht, eine ununterbrochene, durchlässige Folie über ein Trag- und Mitnahmeelement passieren zu lassen; ununterbrochene, elastische Elemente in Berührung mit jeder Folie auf dem genannten Element zu bringen, wobei die Spannung der elastischen Elemente an ihren Kontaktpunkten mit der Folie periodisch geändert wird und die Kontaktpunkte periodisch quer zur Längsrichtung der Folie verschoben werden, um die elastischen Elemente durch Aufkleben auf die Folie derart zu befestigen, daß die elastischen Elemente auf der Folie zwei sich wiederholende, ununterbrochene Profile relativ zur Längsachse der Folie bilden und in den Zonen, die den Endzonen der Höschenwindeln entsprechen, eine schwächere Spannung aufweisen als in den den Schrittzonen der Höschenwindeln entsprechenden Zonen; und Aufbringen von absorbierenden Kissen auf der undurchlässigen Folie, Aufbringen einer für Flüssigkeiten durchlässigen Folie und Aufbringen von adhäsiven Befestigern; und Durchschneiden des so erhaltenen Verbundbandes in Querrichtung zwischen den absorbierenden Kissen,
dadurch **gekennzeichnet,** daß man die periodische Querverschiebung der Kontaktpunkte zwischen den elastischen Elementen und der undurchlässigen Folie so steuert, daß in den Zonen, die den Endzonen der Höschenwindeln entsprechen, sich die genannten beiden Profile periodisch kreuzen oder sich bis auf einen gegenseitigen Abstand einander annähern, der kleiner als 50 % der maximalen Breite der absorbierenden Kissen ist.

11. Verfahren nach Anspruch 10,
dadurch **gekennzeichnet,** daß man die undurchlässige Folie auf ihrer, die elastischen Elemente aufnehmenden Seite im Hinblick auf das Ankleben dieser Elemente auf der Folie schlichtet.

12. Verfahren nach Anspruch 11,
dadurch **gekennzeichnet,** daß man für das Schlichten eine ungeschlichtete Bahn in der Mitte der Breite der undurchlässigen Folie freihält.

13. Verfahren nach Anspruch 11,
dadurch **gekennzeichnet,** daß man das Schlichten der undurchlässigen Folie in einer Zone intermittierend unterbricht, die in der Mitte der Breite der Folie gelegen ist, derart, daß periodisch ein nichtgeschlichtetes Fenster an derjenigen Stelle gebildet wird, an der sich die Wege der elastischen Elemente kreuzen oder berühren.

14. Verfahren nach Anspruch 11,
dadurch **gekennzeichnet,** daß man die undurchlässige Folie ohne Unterbrechung über ihre gesamte Breite schlichtet, und daß man periodisch ein antiadhäsives Material auf die elastischen Elemente aufstäubt.

15. Anlage zur unterbrechungslosen Herstellung von Höschenwindeln nach irgendeinem der Ansprüche 1 bis 9, unter Durchführung des Verfahrens nach irgendeinem der Ansprüche 10 bis 14,
dadurch **gekennzeichnet,** daß sie zum Führen jedes elastischen Elementes eine Führungseinrichtung (13) aufweist, die in unmittelbarer Nähe des Trag- und Mitnahmeelementes (1, 1a), auf dem die undurchlässige Folie (2) zum Aufnehmen der elastischen Elemente (4) läuft, frei um eine im wesentlichen senkrecht zur Oberfläche des genannten Elementes (1, 1a) verlaufende Achse montiert ist, vorzugsweise durch ein Antifriktionslager auf einem Wagen (14), der translatorisch quer zur Verschiebungsrichtung des Trag- und Mitnahmeelementes angetrieben wird.

16. Anlage nach Anspruch 15,
dadurch **gekennzeichnet,** daß der Wagen (14) durch einen Linear- oder Umdrehungsmotor (17) angetrieben wird, der mit Indexierung der Bewegung des Trag- und Mitnahmeelementes (1) durch eine elekronische Steuereinheit (18) betätigt wird, die durch einen Rechner (19) gesteuert wird.

17. Anlage nach Anspruch 15 oder 16,
dadurch **gekennzeichnet,** daß sie zur Erteilung veränderlicher Streckspannungen an die elastischen Elemente drehbare Zuführungsorgane (7) aufweist, die eine Klemmwirkung auf die elastischen Elemente ausüben, deren Bewegung mit veränderlicher Geschwindigkeit durch eine elektronische Steuereinrichtung bewirkt wird, die durch einen Rechner (19) gesteuert wird.

18. Anlage nach Anspruch 16 oder 17,
dadurch **gekennzeichnet,** daß die Positionierungswege für die elastischen Elemente auf der undurchlässigen Folie durch ein Rechnerprogramm trassiert und gesteuert werden, ebenso wie die Form der herausgearbeiteten Schrittausschnitte in der undurchlässigen Folie, beispielsweise durch eine Wasserstrahlvorrichtung.

19. Anlage nach Anspruch 15,
dadurch **gekennzeichnet,** daß die Führungseinrichtung (13) einen Führungskopf mit geschlossenen Haltemitteln für die elastischen Elemente aufweist, beispielsweise in Form von parallelen Bohrungen oder Kanälen, in die die elastischen Elemente gefädelt sind.

20. Anlage nach irgendeinem der Ansprüche 15 bis 19,
dadurch **gekennzeichnet,** daß das Trag- und Mitnahmeelement aus einer Drehtrommel (1) besteht.

21. Anlage nach irgendeinem der Ansprüche 15 bis 19,
dadurch **gekennzeichnet,** daß das Trag- und Mitnahmelement aus einem saugenden Transportband (1a) besteht.
